# EUROPÄISCHE PATENTSCHRIFT

(11) **EP 2 512 993 B1**
(45) Veröffentlichungstag und Bekanntmachung des Hinweises auf die Patenterteilung: **27.11.2013**
(21) Anmeldenummer: 10796007.2
(22) Anmeldetag: 13.12.2010
(51) Int. Cl.: C07C 201/08, C01B 21/40, C02F 9/00

(54) **VERFAHREN ZUR AUFARBEITUNG VON NOx-HALTIGEN ABGASEN AUS ABWASSERSTRÖMEN VON NITRIERANLAGEN**
METHOD FOR TREATING NOX-CONTAINING EXHAUST GAS FROM PROCESS WASTEWATER FROM NITRATION FACILITIES
PROCÉDÉ DE RETRAITEMENT DE GAZ D'ÉCHAPPEMENT CONTENANT DES NOx ISSUS DES FLUX D'EAUX USÉES D'INSTALLATIONS DE NITRURATION

(30) Priorität: 16.12.2009 EP 09179507
(43) Veröffentlichungstag der Anmeldung: 24.10.2012
(73) Patentinhaber: BASF SE, 67056 Ludwigshafen (DE)
(72) Erfinder: FRITZ, Rüdiger, 02994 Bernsdorf (DE); HEMPEL, Renate, 01945 Ruhland (DE); ZÖLLINGER, Michael, 73054 Eislingen (DE); ALLARDT, Holger, 01987 Schwarzheide (DE); REETZ, Reiner, 01987 Schwarzheide (DE)
(86) Internationale Anmeldenummer: PCT/EP2010/069530
(87) Internationale Veröffentlichungsnummer: WO 2011/082977

(56) Entgegenhaltungen:
- EP-A1- 1 493 730
- EP-A2- 0 005 203
- EP-A2- 1 178 017
- WO-A1-2005/037766
- WO-A1-2009/027416
- US-B1- 6 245 242

## Beschreibung

Die vorliegende Erfindung betrifft ein Verfahren zur Aufarbeitung von Nitrit enthaltenden alkalischen Prozessabwassern aus der Nitrierung von aromatischen Verbindungen, wobei das alkalische Prozessabwasser durch Zugabe von Säuren angesäuert wird und das aus dem angesäuerten Prozessabwasser entweichende, Stickoxide enthaltende Abgas aufgearbeitet wird.

Aromatische Nitroverbindungen wie Mono- und Dinitrotoluol werden üblicherweise durch Nitrierung der entsprechenden aromatischen Verbindungen mittels einer Mischung aus konzentrierter Salpetersäure und konzentrierter Schwefelsäure, die als Nitriersäure bezeichnet wird, hergestellt. Dabei entsteht eine organische Phase, die das Rohprodukt der Nitrierung enthält, und eine wässrige Phase, die im Wesentlichen Schwefelsäure, Reaktionswasser und durch die Nitriersäure eingetragenes Wasser enthält. Die Salpetersäure wird fast vollständig bei der Nitrierung verbraucht.

Nach Trennung der beiden Phasen wird die wässrige, schwefelsäurehaltige Phase je nach Technologie des Nitrierverfahrens direkt oder nach Aufkonzentrierung wieder mit frischer Salpetersäure gemischt und zur Nitrierung eingesetzt. Mindestens ein Teil der Schwefelsäure muss jedoch kontinuierlich oder diskontinuierlich aus dem Gesamtprozess ausgeschleust werden, um eine Aufkonzentration von Verunreinigungen, insbesondere metallischer Salze, zu vermeiden (siehe auch DE 10 143 800 C1). Bei den Verunreinigungen handelt es sich beispielsweise um ursprünglich in der Salpetersäure enthaltene Verunreinigungen und um Metallverbindungen, die unter den bei der Reaktion und der Aufarbeitung der wässrigen Phase herrschenden, stark korrosiven Bedingungen aus den Reaktor- und Rohrmaterialen herausgelöst werden.

Bei der Aufkonzentrierung der bei der Nitrierung anfallenden wässrigen schwefelsäurehaltigen Phase werden ein wässriges Destillat mit niedrigem Schwefelsäuregehalt, im Folgenden als wässriges Destillat der Schwefelsäureaufkonzentration bezeichnet, und eine Phase mit hohem Schwefelsäuregehalt, im Folgenden aufkonzentrierte Schwefelsäure genannt, erhalten. Der aus dem Nitrierverfahren ausgeschleuste Teil der aufkonzentrierten Schwefelsäure wird im Folgenden auch Abfallschwefelsäure genannt.

Das Rohprodukt der Nitrierung von aromatischen Verbindungen wie Benzol, Toluol, Xylol, Chlorbenzol etc. zu den entsprechenden Nitroaromaten enthält üblicherweise neben den gewünschten Nitroaromaten wie Nitrobenzol (NB) und Dinitrobenzol (DNB), Mono- und Dinitrotoluol (MNT und DNT), Nitrochlorbenzol (NCB) oder Nitroxylol auch geringe Mengen Mono-, Di- und Trinitrophenole (im Folgenden Nitrophenole genannt), Mono-, Di- und Trinitrokresole (im Folgenden Nitrokresole genannt) und Mono-, Di- und Trinitroxylenole (im Folgenden Nitroxylenole genannt) und andere Hydroxylgruppen und Nitrogruppen enthaltende Verbindungen sowie Mono- und Dinitrobenzoesäuren (im Folgenden Nitrobenzoesäuren genannt).

Aromatische Nitroverbindungen, die keine Hydroxylgruppe oder Carboxylgruppe im Molekül enthalten, werden im Rahmen der Erfindung auch als neutrale Nitrokörper oder neutrale Nitroaromaten bezeichnet. Nitrophenole, Nitrokresole, Nitroxylenole und Nitrobenzoesäuren werden im Folgenden auch als Hydroxynitroaromaten zusammengefasst.

Das Rohprodukt aus der Nitrierung muss vor Weiterverwendung von den unerwünschten Nebenprodukten befreit werden. Üblicherweise werden die Nebenprodukte nach Abtrennung der Nitriersäure durch mehrstufige Wäsche mit saurer, alkalischer und neutraler Waschflüssigkeit abgetrennt, wobei in der Regel in der aufgeführten Reihenfolge gewaschen wird. Die alkalische Wäsche wird üblicherweise mit wässriger Natronlauge, wässriger Natriumcarbonatlösung oder wässriger Ammoniaklösung durchgeführt. Das dabei entstehende alkalische Prozessabwasser enthält Nitrophenole, Nitrokresole, Nitroxylenole und Nitrobenzoesäuren in Form ihrer wasserlöslichen Salze der eingesetzten Base. Sie liegen üblicherweise in einer Konzentration von 0,2 bis 2,5 Gew.-%, bezogen auf das alkalische Prozessabwasser vor. Das alkalische Prozessabwasser enthält auch bei der Nitrierung gebildete neutrale Nitrokörper, insbesondere Reaktionsprodukte. Neutrale Nitrokörper sind im alkalischen Prozessabwasser üblicherweise in einer Menge von mehreren 1000 ppm enthalten. Das alkalische Prozessabwasser enthält in der Regel 500 bis 5000 ppm Nitrate, 500 bis 5000 ppm Nitrit und einige Hundert ppm Sulfat. Diese Ionen stammen aus der Nitrierung. Aus den Inhaltsstoffen ergibt sich ein chemischer Sauerstoffbedarf von 1000 bis 20000 mg/L.

Die Nitrophenole, Nitrokresole, Nitroxylenole, Nitriobenzoesäuren und vor allem ihre Salze sind intensiv gefärbt und stark umwelttoxisch. Außerdem sind die Nitrophenole und speziell ihre Salze in höheren Konzentrationen oder in Substanz Sprengstoffe und müssen vor Abgabe des Abwassers aus diesem entfernt und derart entsorgt werden, dass von ihnen keine Gefahr für die Umwelt ausgeht. Das alkalische Prozessabwasser enthält auch bei der Nitrierung gebildete neutrale Nitrokörper, insbesondere Reaktionsprodukte. Da die aromatischen Nitroverbindungen insgesamt bakterizide Eigenschaften aufweisen und somit eine biologische Reinigung des Abwassers unmöglich machen, ist eine Reinigung oder Aufarbeitung der aromatische Nitroverbindungen enthaltenden Abwässer notwendig.

Zur Entfernung der Nitrophenole, Nitrokresole, Nitroxylenole, Nitrobenzoesäuren und der neutralen Nitroaromaten aus den Prozessabwässern sind zahlreiche Verfahren in der Literatur beschrieben, beispielsweise Extraktion, Adsorption, Oxydation oder Thermolyse.

In der Encyclopedia of Chemical Technology, Kirk-Othmer, Fourth Edition 1996, Vol. 17, S. 138 wird ein Extraktionsverfahren zur Abtrennung von Nitrobenzol beschrieben, bei dem das im Abwasser bei der entsprechenden Temperatur gelöste Nitrobenzol durch Extraktion mit Benzol entfernt wird. Benzol, das sich im Wasser gelöst hat, wird vor der Endbehandlung des Abwassers durch Strippen entfernt.

Gemäß US-A 6,506,948 werden die bei der Nitrierung von Toluol anfallenden wässrigen Waschphasen direkt mit Toluol extrahiert, wobei jeder der entstehenden Abwasserströme separat extrahiert wird. Der Toluolstrom wird anschließend in den Nitrierprozess geführt und umgesetzt. Dabei bleiben Nitrokresole und Nitrobenzoesäuren im alkalischen Abwasserstrom gelöst und müssen anschließend separat entfrachtet werden.

In EP 0 005 203 wird ein thermisches Verfahren zur Aufarbeitung von Hydroxynitroaromaten enthaltenden Abwässern beschrieben. Hierbei werden die Abwässer, die die Hydroxynitroaromaten in Form ihrer wasserlöslichen Salze enthält, unter Luft- und Sauerstoffausschluss unter Druck auf Temperaturen im Bereich 150 bis 500°C erhitzt.

Aus EP 0 953 546 ist ein thermisches Verfahren zur Aufarbeitung von Abwasserströmen aus Nitrieranlagen, bei dem gleichzeitig Hydroxynitroaromaten und neutrale Nitroaromaten abgebaut werden können, bekannt.

WO 2005/037766 offenbart ein Verfahren zur Entfernung von Nitrokresolen aus Abwasser der Mononitrotoluolherstellung durch ansäuern alkalischen Abwassers zu einem pH von höchstens 3.

EP 1 178 017 offenbart die Entfernung von Nitriten aus Industrieabwässern. Das Abwasser wird in diesem Verfahren über einen porösen Katalysator geleitet, an dem Bakterien haften und wobei Wasserstoff als Reduktionsmittel eingesetzt wird.

Gemäß WO 2009/027416 A1 werden vor der thermolytischen Behandlung der alkalischen Abwässer aus der Nitrierung die darin gelösten, aromatischen Nitroverbindungen, die keine Hydroxylgruppen enthalten, durch Extraktion entfernt.

Die gelösten Nitroaromaten und Hydroxynitroaromaten können weiterhin im sauren Milieu durch Extraktion mit einem organischen Lösungsmittel entfernt werden (Ullmanns Enzyklopädie der technischen Chemie, 4. Auflage, Band 17, Seite 386).

Die in dem alkalischen Prozessabwasser enthaltenen Hydroxynitroaromaten können auch durch Ansäuern in eine sich abscheidende organische Phase überführt werden, die anschließend abgetrennt wird. Um das Auskristallisieren der Hydroxynitroaromaten zu verhindern, muss die für das Abscheiden und Abtrennen verwendete Apparatur beheizt werden. Dennoch tritt das Problem des so genannten "Foulings" auf. Dies bedeutet, dass die zur Abtrennung der sich ausscheidenden organischen Phase verwendeten Pumpen und Rohrsysteme sehr schnell durch ausfallende und auskristallisierende Verunreinigungen verstopfen und daher ein hoher Reinigungsbedarf besteht.

Ein derartiges Verfahren ist in EP 1 493 730 A1 beschrieben. Gemäß diesem Verfahren werden die Abwasserströme der sauren und alkalischen DNT-Wäschen und der Schwefelsäureaufkonzentrierung vermischt, so dass sich ein pH-Wert unterhalb von 5 einstellt. Bei dem Abwasser der Schwefelsäureaufkonzentrierung handelt sich um das Destillat der Schwefelsäureaufkonzentrierung mit einer Schwefelsäurekonzentration von 0,2 bis 1 Gew.- %. Beim Ansäuern scheidet sich eine organische Phase ab, die abgetrennt wird. Die wässrige Phase wird separat einer weiteren Abwasseraufbereitung zugeführt.

Weiterhin ist bekannt, bei der Dinitrotoluol-Herstellung das dinitrotoluolhaltige, alkalische Abwasser durch eine Absorption am Klärschlamm vom größten Teil des bei 70°C gelösten 2,4- und 2,6-Dinitrotoluol zu befreien, bevor die Hydroxynitroaromaten mittels Ozonisierung aufgeschlossen werden. Hierbei stellt sich das Problem, dass die Nitrokresole durch die Ozonierung nicht umgewandelt werden können. Zudem enthält das alkalische Abwasser noch Nitrit, das erst durch Ozon zu Nitrat oxidiert wird und so zu einem erhöhten Ozonbedarf führt. So werden bei einer Konzentration von 3000 mg/L Nitrit im alkalischen Prozessabwasser etwa 25 bis 40% des Ozons für die Nitrit-Oxidation verbraucht.

Daher besteht die Aufgabe der vorliegenden Erfindung darin, ein Verfahren zur Aufarbeitung von alkalischen Prozessabwassern der Nitrierung von aromatischen Verbindungen bereitzustellen, bei dem anfallende Neben- und Abfallprodukte der Nitrierung von aromatischen Verbindungen im Verfahren wiederverwertet können und/oder mit einer geringen Belastung an unerwünschten Bestandteilen der anderen weiteren Verfahren der Abwasserentsorgung bzw. -aufarbeitung angeführt werden können.

Diese Aufgabe wird gelöst durch das folgende Verfahren zur Aufarbeitung von alkalischen Prozessabwassern aus der Nitrierung von aromatischen Verbindungen zu Mono-, Di- und Trinitroaromaten, umfassend die Schritte
a) Ansäuern des Prozessabwassers durch Zugabe von Säure auf einen pH-Wert unterhalb von 5, wobei eine sich abscheidende organische Phase, eine saure wässriger Phase und eine gasförmige NOₓ-haltige Phase entstehen, und
b) Abführen der gasförmigen, NOₓ-haltige Phase.

Beim Ansäuern des alkalischen Prozessabwassers entsteht aus den darin enthaltenen Nitriten Salpetrige Säure, die unter den sauren Bedingungen instabil ist und sich zu NO, NO₂ und Salpetersäure zersetzt. Die entstehenden Stickoxide NO und NO₂ können wie im "Lehrbuch der Anorganischen Chemie", Holleman-Wiberg, 101. Auflage 1995, S. 690-710 beschrieben beispielsweise weiter zu höheren gasförmigen Stickstoffoxiden wie N₂O₃ und N₂O₄ oder unter Dimerisierung zu N₂O₂ reagieren. Die Stickstoffoxide und deren höhere Homologe werden im Weiteren als NOₓ bezeichnet.

Das mit dem erfindungsgemäßen Verfahren behandelte alkalische Prozessabwasser weist eine deutlich niedrigere Nitritkonzentration aus. Dies erleichtert die weitere Aufarbeitung der wässrigen Phase.

Typischerweise beträgt die prozentuale Erniedrigung des Gehalts an Nitrit in einem erfindungsgemäß behandelten alkalischen Prozessabwasser aus einer Anlage zur Herstellung von Dinitrotoluol in der Summe (Nitrit) 90 - 99%.

Die sich abscheidende organische Phase enthält den größten Teil der im alkalischen Prozessabwasser enthaltenden neutralen Nitroaromaten und Hydroxynitroaromaten, die säure wässrige Phase ist an diesen Verbindungen stark verarmt.

Das NOₓ-haltige Abgas kann nun auf unterschiedlichen Wegen entsorgt werden, beispielsweise durch Verbrennung mit nach geschalteten Katalysatoren zur Abgasreinigung oder durch alkalische Absorption des aus der Ansäuerung entstandenen Abgasstroms bspw. mit NaOH oder anderen alkalischen Wäschern erfolgen. Bei der alkalischen Absorption ist besonders nachteilig, dass CO₂, welches ebenfalls im Abgasstrom enthalten sein kann, ebenfalls absorbiert wird und sich die Menge an benötigtem Alkali erhöht.

In einer bevorzugten Ausführungsform werden die in der abgeführten Phase enthaltenen NOₓ wieder der Salpetersäureherstellung zugeführt. Besonders rohstoffsparend ist, wenn die so erhaltene Salpetersäure wieder bei der Nitrierung der aromatischen Verbindungen eingesetzt wird, da die NOₓ dem Nitrierverfahren auf diese Weise nicht verloren gehen.

Gemäß einer weiteren bevorzugten Ausführungsform des erfindungsgemäßen Verfahrens wird für das Ansäuern des alkalischen Prozessabwassers die der bei der Aufkonzentrierung anfallende Schwefelsäure aus dem Herstellungsprozess der Nitroaromaten verwendet. Dies ist insbesondere von Vorteil, da ein Teil der aufkonzentrierten Schwefelsäure als so genannte Abfallschwefelsäure aus dem Kreislauf von Nitrierung und Schwefelsäureaufarbeitung sowieso ausgeschleust und entsorgt werden muss. Die aufkonzentrierte Schwefelsäure enthält die durch Korrosion (Rohrleitungen) bei der Nitrierung anfallenden Salze umfassend Fe, Cr, Ni, Ta, und Spuren weiterer Schwermetalle in Form ihrer Sulfate. Üblicherweise wird bei einem Anstieg der Salzkonzentration über 300 ppm ein Teil der Säure als so genannte Abfallschwefelsäure aus dem Prozess geschleust und muss entsorgt bzw. mit anderen Verfahren aufgereinigt werden. Somit ist die Verwendung dieser Abfallschwefelsäure besonders vorteilhaft. Überraschenderweise hat sich gezeigt, dass ein Teilstrom der auszuschleusenden Menge an aufkonzentrierter Schwefelsäure im erfindungsgemäßen Verfahren eingesetzt werden kann, ohne dass eine weitere Zugabe zusätzlicher Säure erforderlich ist. Dies führt zu einem sehr ökonomischen Einsatz der unterschiedlichen Stoffströme.

Das erfindungsgemäße Verfahren wird zur Aufarbeitung von alkalischem Prozessabwasser aus der Nitrierung von aromatischen Verbindungen eingesetzt. Vorzugsweise wird das Verfahren bei der Nitrierung von Benzol, Toluol, Xylol, Chlorbenzol und/oder Dichlorbenzol eingesetzt.

Das aus der ein- bzw. mehrstufigen Wäsche des Rohprodukts der Nitrierung mit wässriger, alkalischer Lösung wie Natronlauge, wässriger Carbonat- oder Hydrogencarbonatlösung oder wässriger Ammoniaklösung erhaltene alkalische Prozessabwasser weist in Abhängigkeit von der eingesetzten Base einen pH-Wert von 7,5 bis 13, bevorzugt 8 bis 10, gemessen bei 60°C auf.

Erfindungsgemäß wird das alkalische Prozessabwasser in Schritt a) durch Zugabe von Säure auf einen pH-Wert unterhalb von 5, bevorzugt von 0,1 bis 1 eingestellt. Die pH-Wert-Angaben beziehen sich jeweils auf die Messung bei 60°C. Bevorzugt wird zum Ansäuern konzentrierte Säure eingesetzt mit einer Säurekonzentration von 70 bis 95 Gew.-%, besonders bevorzugt wird Schwefelsäure verwendet.

Gemäß einer bevorzugten Ausführungsform der Erfindung wird zum Ansäuern in Schritt a) Schwefelsäure aus der Aufarbeitung der bei der Nitrierung anfallenden wässrigen, schwefelsäurehaltigen Phase, besonders bevorzugt die aufkonzentrierte Schwefelsäure eingesetzt. Die zur Ansäuerung eingesetzte aufkonzentrierte Schwefelsäure weist eine Konzentration von 85 bis 95 Gew.-%, bevorzugt von 90 bis 93 Gew.-% auf. Gemäß einer bevorzugten Ausführungsform wird nur bei der Nitrierung anfallende Abfallschwefelsäure zum Ansäuern in Schritt a) zugegeben, besonders bevorzugt wird die gesamte bei der Nitrierung anfallende Abfallschwefelsäure in Schritt a) zugegeben. Die Steuerung der Zugabe der aufkonzentrierten Schwefelsäure erfolgt in vorteilhafter Weise über eine Online-pH-Messung.

Erfindungsgemäß bevorzugt wird das alkalische Prozeßabwasser bei Temperaturen von 20 bis 90°C, besonders bevorzugt bei Temperaturen von 55 bis 70°C angesäuert. Beim Ansäuern des alkalischen Prozessabwassers in Schritt a) scheidet sich eine organische Phase ab, die Hydroxynitroaromaten, Nitrobenzoesäuren und neutrale Nitrokörper enthält, und aus der wässrigen sauren Phase entweichen während und auch noch nach Erreichen des Ziel-pH-Werts NOₓ. Für den Fall, dass als alkalische Waschflüssigkeit wässrige Alkalicarbonat- oder Alkalihydrogencarbonatlösung verwendet wurde, entweichen zusätzlich große Mengen CO₂. Die sich abscheidende gasförmige Phase enthält bei vorangegangener DNT-Wäsche mit wässriger Alkalicarbonat- oder Alkalihydrogencarbonatlösung üblicherweise 70 bis 98,9 Vol.-% Kohlendioxid und 1,1 bis 30 Vol.-% nitrose Gase (NO, NO₂, N₂O) bzw. NOₓ. Bevorzugt enthält das sich abscheidende Gasgemisch 80 bis 98 Vol.-% Kohlendioxid und 2 bis 20 Vol.-% nitrose Gase bzw. NOₓ.

Enthält das Prozessabwasser anstatt Alkalicarbonat oder Alkalihydrogencarbonat eine oder mehrere andere Basen, die nach dem Ansäuern keine gasförmige Komponenten bilden, besteht die gasförmige Phase im Wesentlichen aus NOₓ, üblicherweise 47 bis 98% Stickstoffmonoxid, 1 bis 47% Stickstoffdioxid und 1 bis 6% Distickstoffmonoxid.

Gemäß einer bevorzugten Ausführungsform werden die NOₓ durch ein Inertgas aus der sauren wässrigen und der organischen Phase ausgestrippt, d. h. Inertgas wird durch die beiden Phasen durchgeleitet und nimmt die darin absorbierten/gelösten NOₓ mit. Das Inertgas wird bevorzugt ausgewählt aus Stickstoff, Mischungen aus Stickstoff und Sauerstoff, Luft und/oder Kohlendioxid.

Beim erfindungsgemäßen Verfahren hat sich als besonders vorteilhaft erwiesen, wenn das alkalische Prozessabwasser aus der alkalischen Wäsche mit wässrigen Lösungen von Alkalicarbonat und/oder Alkalihydrogencarbonat stammt. Beim Ansäuern des mit diesen Basen alkalisierten Prozessabwassers entsteht neben der bereits beschriebenen gasförmigen NOₓ auch eine große Menge an gasförmigem CO₂, welches gleichzeitig eine Strippung von NOₓ aus dem sauren Prozessabwassers gewährleistet. Diese Ausführungsform ist besonders vorteilhaft, weil eine zusätzliche Strippung mit Inertgas entfallen kann.

In Schritt b) wird die gasförmig NOₓ enthaltende Phase abgeführt, d. h. von der organischen und der sauren wässrigen Phase abgetrennt. Dies wird vorzugsweise von der Weiterbehandlung der organischen und der wässrigen Phase durchgeführt.

Nach Schritt b) werden die in der abgeführten gasförmigen Phase enthaltenen NOₓ gemäß einer bevorzugten Ausführungsform in Schritt c) zu Salpetersäure weiterverarbeitet. Das erfindungsgemäße Verfahren zur Aufarbeitung von alkalischen Prozessabwassern aus der Nitrierung von aromatischen Verbindungen zu Mono-, Diund Trinitroaromaten umfasst in dieser Ausführungsform die folgenden Schritte
a) Ansäuern des Prozessabwassers durch Zugabe von Säure auf einen pH-Wert unterhalb von 5, wobei eine sich abscheidende organische Phase, eine saure wässriger Phase und eine gasförmige NOₓ-haltige Phase entstehen, und
b) Abführen der gasförmigen, NOₓ-haltigen Phase,
c) Weiterverarbeiten der NOₓ, die in der in Schritt b) abgeführten gasförmigen Phase enthalten sind, zu Salpetersäure.

Bevorzugt wird dabei die im Schritt b) abgeführte NOₓ-haltige Phase in Schritt c) ohne vorherige Reinigung und Abtrennung von anderen Gasbestandteilen direkt einer Absorptionskolonne einer Salpetersäureherstellung zugeführt.

Die in Schritt c) enthaltene Salpetersäure wird bevorzugt in die Nitrierung der aromatischen Verbindungen zurückgeführt. Gemäß einer besonders bevorzugten Ausführungsform des erfindungsgemäßen Verfahrens wird die NOₓ-haltige Phase in die Absorptionskolonnen der NOₓ-Absorption der Salpetersäurerückgewinnung der Nitrieranlage eingespeist. Besonders vorteilhaft ist, wenn die gesamte, in Schritt b) abgeführte NOₓ-haltige Phase direkt und ohne Reinigung und vorherige Abtrennung von ggf. enthaltendem CO₂ rückgeführt wird.

Die Rückführung erfolgt dabei bevorzugt auf eine Absorberkolonne, wie sie für die Aufarbeitung der NOₓ-Abgase aus der Schwefelsäureaufkonzentrierung, beispielsweise nach dem Verfahren DE 10 143 800 C1, installiert ist. Dabei wird unter einem Druck von 6 bar und Temperaturen von 10 - 60 °C aus NOₓ bei Gegenstrom von Wasser Salpetersäure mit einer Konzentration von etwa 60 Gew.-% hergestellt, welche anschließend für die Nitrierung von aromatischen Verbindungen zur Verfügung steht und auf diese Weise dem Prozess der Nitrierung zurückgeführt werden kann. Das mitgeführte CO₂ verhält sich unter den herrschenden Bedingungen inert und kann über den Kopf der Kolonne abgeführt werden.

Nach Schritt b) kann die abgeführte NOₓ-haltige Phase auch weiteren, dem Fachmann bekannten Verfahren zur Entsorgung von NOₓ, beispielsweise einer Verbrennung zugeführt werden.

Die in Schritt a) erhaltene wässrige und organische Phase können weiter aufgearbeitet werden. Gemäß einer Ausführungsform wird die organische Phase abgetrennt und die saure wässrige Phase mit einem Lösungsmittel extrahiert. Als Lösemittel sind dabei die aromatischen Verbindungen, die als Ausgangsprodukte bei der Nitrierung eingesetzt werden, bevorzugt.

Gemäß einer weiteren Ausführungsform der Erfindung wird nach Abtrennung der organischen Phase die wässrige Phase, gegebenenfalls nach Extraktion mit einem organischen Lösungsmittel, einer Ozonolyse, einer Thermolyse oder einer biologischen Abwasserreinigung zugeführt.

Das erfindungsgemäße Verfahren kann batchweise oder kontinuierlich durchgeführt werden. Erfindungsgemäß bevorzugt wird das Verfahren kontinuierlich durchgeführt.

Im Folgenden wird das erfindungsgemäße Verfahren anhand von Beispielen näher erläutert:

### Beispiel 1

In einem 1000 mL Rührbehälter mit Gasabführung, Innenthermometer und pH-Elektrode werden 500 mL eines alkalischen Prozessabwassers aus der Wäsche mit wässriger Natriumcarbonatlösung aus der Produktion von Dinitrotoluol bei 60 °C vorgelegt. Anschließend wird der gesamte Innenraum mit Stickstoff gespült und vollständig inertisiert. Die Konzentrationen an Carbonat und Nitrit im Prozessabwasser sind in Tabelle 1 aufgeführt. Der pH-Wert des alkalischen Prozessabwassers betrug 9. Anschließend wird das Prozessabwasser mit 93%iger aufkonzentrierter Abfallschwefelsäure versetzt, bis ein pH-Wert von 1 erreicht wird und 5 Minuten gerührt. Das entstehende Gasgemisch wird über die Gasabführung in eine benachbarte Absorberkaskade geleitet, wobei die ersten beiden Flaschen mit einer genau eingestellten angesäuerten 0.02 m Kaliumpermanganatlösung (zur NO-Bestimmung), die beiden letzten Flaschen mit einer 0,1 m NaOH (zur CO₂-Bestimmung) gefüllt waren. Anschließend wurde das Totvolumen der Apparatur gründlich mit Stickstoff gespült (nicht gestrippt), um möglichst viel entstandenes Gas in die Absorberkolonnen zu leiten. Danach wurde die NaOH-Lösungen vereinigt und potentiometrisch der Carbonatgehalt mit 0,1 m HCl bestimmt und auf diese Weise die CO₂-Menge zurückgerechnet. Die Kaliumpermanganatlösungen wurden nach Vereinigung anschließend mit Oxalsäure titriert und die Stickstoffmonoxidmenge bestimmt. Die bestimmten Gasmengen sind in Tabelle 1 dargestellt. Die entstandene Gasmenge (berechnet aus NO und CO₂) betrug 3,25 l.

**Tabelle 1:**

| Komponente | Abwassergemisch Ausgangswerte | Abwassergemisch nach Ansäuern | Gaszusammensetzung | Gasmenge |
|---|---|---|---|---|
| Carbonat | 1,60 % | n. n.* | 86 Vol% CO₂ | 2,795 l |
| Nitrit | 0,30 % | n. n.* | 14 Vol% NO | 0,455 l |

| | | | | |
|---|---|---|---|---|
| * nicht nachweisbar (ionenchromatographisch) | | | | |

### Beispiel 2

In einem 1000 mL Rührbehälter mit Gasabführung, Innenthermometer und pH-Elektrode werden 500 mL 1,0 m NaOH mit 0,3% NaNO₂ bei 60 °C vorgelegt. Anschließend wird der gesamte Innenraum mit Stickstoff gespült und vollständig inertisiert. Anschließend wird die Lösung mit 93%iger aufkonzentrierter Abfallschwefelsäure versetzt, bis ein pH-Wert von 1 erreicht wird und 5 Minuten gerührt. Das entstehende Gasgemisch wird über die Gasabführung in eine benachbarte Absorberkaskade, gefüllt mit einer genau eingestellten angesäuerten 0.02 m Kaliumpermanganatlösung, geleitet. Anschließend wurde das Totvolumen der Apparatur gründlich mit Stickstoff gespült (nicht gestrippt), um möglichst viel entstandenes Gas in die Absorberkolonnen zu leiten. Danach wurde die Kaliumpermanganatlösungen nach Vereinigung mit Oxalsäure titriert und die NO Menge bestimmt.

Anschließend wurde der gleiche Versuch durchgeführt mit dem Unterschied, dass das Spülen der Apparatur mit Stickstoff unterspieglig (Strippung) erfolgte. In Tabelle 2 sind die Ergebnisse nach jeweils 5minütigem Spülen mit 50 l/h Stickstoff aufgeführt.

**Tabelle 2: NaOH/NO₂-Gemisch Einfluss des Strippens**

| Strippung | Ausganswerte für Nitrit | Entstandene Gasmenge (berechnet aus NO) |
|---|---|---|
| nein | 0,30 % | 0,260 l |
| ja | 0,30% | 0,455 l |

Das Strippen (Durchspülen) der angesäuerten Lösung führt zu einer höheren Ausbeute an ausgetriebenen NO.

Im Vergleich mit Beispiel 1 zeigt sich auch, dass das bei Verwendung von wässriger Natriumhydrogencarbonatlösung als alkalische Waschflüssigkeit entstehende CO₂ ein wirksames Mittel zum Strippen ist.

### Beispiel 3

In einen 50 L Rührbehälter mit Gasabführung, Prozessabwasserzuführung, Säuredosierung, pH-Elektrode und Prozessabwasserabführung wurden kontinuierlich 220 l/h alkalisches Prozessabwasser zugeführt und kontinuierlich mit Abfallschwefelsäure (93%ig) auf einen pH-Wert von 1 eingestellt. Das angesäuerte Prozessabwasser wurde kontinuierlich ca. 220 l/h (füllstandsgeregelt) einer Weiterbehandlung zugeführt (Rührzellenextraktor, pulisierende Kolonne, Mixer-Settler). Das entstehende Gasgemisch wurde über eine Leitung einem Verdichter und schließlich einer Absorberkolonne, welche für die Aufarbeitung der NOx-Abgase aus der Schwefelsäureaufkonzentrierung nach dem Verfahren DE 101 43 800 C1 installiert ist, zugeführt.

### Beispiel 4

In einen 50 L Rührbehälter mit Gasabführung, Prozessabwasserzuführung, Säuredosierung, pH-Elektrode und Prozessabwasserabführung wurden kontinuierlich 220 l/h alkalisches Prozessabwasser zugeführt und kontinuierlich mit Abfallschwefelsäure (93%ig) auf einen pH-Wert von 1 eingestellt. Das angesäuerte Prozessabwasser wurde kontinuierlich ca. 220 l/h (füllstandsgeregelt) einer Weiterbehandlung zugeführt (Rührzellenextraktor, pulisierende Kolonne, Mixer-Settler). Das entstehende Gasgemisch wurde über eine Leitung und ohne weitere Verdichtung einem Verbrennungsofen mit angeschlossener Rauchgasreinigung zugeführt.

## Patentansprüche

1. Verfahren zur Aufarbeitung von alkalischen Prozessabwassern aus der Nitrierung von aromatischen Verbindungen zu Mono-, Di- und Trinitroaromaten, umfassend die Schritte
a) Ansäuern des Prozessabwassers durch Zugabe von Säure auf einen pH-Wert unterhalb von 5, wobei eine sich abscheidende organische Phase, eine saure wässriger Phase und eine gasförmige NOₓ-haltige Phase entstehen, und
b) Abführen der gasförmigen NOₓ-haltigen Phase.

2. Verfahren nach Anspruch 1, **dadurch gekennzeichnet, dass** zum Ansäuern in Schritt a) Schwefelsäure aus der Aufarbeitung der bei der Nitrierung anfallenden wässrigen schwefelsäurehaltigen Phase eingesetzt wird.

3. Verfahren nach Anspruch 1 oder 2, **dadurch gekennzeichnet, dass** zum Ansäuern in Schritt a) aufkonzentrierte Schwefelsäure aus der Aufarbeitung der bei der Nitrierung anfallenden wässrigen schwefelsäurehaltigen Phase eingesetzt wird.

4. Verfahren nach einem der Ansprüche 1 bis 3, **dadurch gekennzeichnet, dass** die beim Ansäuern in Schritt a) entstehenden NOₓ durch ein Inertgas aus der organischen und der sauren wässrigen Phase ausgestrippt und in die gasförmige Phase überführt werden.

5. Verfahren nach einem der Ansprüche 1 bis 4, **dadurch gekennzeichnet, dass** die beim Ansäuern in Schritt a) NOₓ durch Stickstoff, Mischungen aus Stickstoff und Sauerstoff, Luft und/oder Kohlendioxid aus der organischen und der sauren wässrigen Phase ausgestrippt und in die gasförmige Phase überführt werden.

6. Verfahren nach einem der Ansprüche 1 bis 5, **dadurch gekennzeichnet, dass** das alkalische Prozessabwasser aus der Wäsche des Rohprodukts aus der Nitrierung mit wässriger Alkalicarbonat- und/oder Alkalihydrogencarbonatlösung stammt und die Stickoxide durch das bei der Ansäuerung des alkalischen Prozessabwassers entstehende Kohlendioxid aus der organischen und der sauren wässrigen Phase ausgestrippt und in die gasförmige Phase überführt werden.

7. Verfahren nach einem der Ansprüche 1 bis 6, **dadurch gekennzeichnet, dass** es sich bei der zu nitrierenden aromatischen Verbindung um Benzol, Toluol, Xylol, Chlorbenzol und/oder Dichlorbenzol handelt.

8. Verfahren nach einem der Ansprüche 1 bis 7, **dadurch gekennzeichnet, dass** das alkalische Prozessabwasser bei Temperaturen von 20 bis 90 °C, bevorzugt von 55 bis 70 °C angesäuert wird.

9. Verfahren nach einem der Ansprüche 1 bis 8, **dadurch gekennzeichnet, dass** Schritt b) vor der Weiterbehandlung der organischen und der wässrigen Phase durchgeführt wird.

10. Verfahren nach einem der Ansprüche 1 bis 9, **dadurch gekennzeichnet, dass** die saure wässrige Phase nach Abtrennen der organischen Phase mit einem Lösungsmittel extrahiert wird.

11. Verfahren nach einem der Ansprüche 1 bis 10, **dadurch gekennzeichnet, dass** die saure wässrige Phase nach Abtrennen der organischen Phase, ggf. nach Extraktion mit einem Lösungsmittel, einer Ozonolyse, einer Thermolyse und/oder einer biologischen Abwasserreinigung zugeführt wird.

12. Verfahren nach einem der Ansprüche 1 bis 11, **dadurch gekennzeichnet, dass** nach Schritt b) Schritt c) durchgeführt wird, wobei in Schritt c) die in der abgeführten gasförmigen Phase enthaltenen NOₓ zu Salpetersäure weiterverarbeitet werden.

13. Verfahren nach Anspruch 12, **dadurch gekennzeichnet, dass** die abgeführte NOₓ -haltige Phase in Schritt c) ohne vorherige Reinigung und Abtrennung von anderen Gasbestandteilen direkt einer Absorptionskolonne einer Salpetersäureher-stellung zugeführt wird.

14. Verfahren nach Anspruch 12 oder 13, **dadurch gekennzeichnet, dass** die in Schritt c) erhaltene Salpetersäure in die Nitrierung der aromatischen Verbindungen zurückgeführt wird.

15. Verfahren nach einem der Ansprüche 1 bis 11, **dadurch gekennzeichnet, dass** nach Schritt b) die abgeführte NOₓ-haltige Phase einer Verbrennung zugeführt wird.

## Claims

1. A process for workup of alkaline process wastewaters from the nitration of aromatic compounds to mono-, di- and trinitroaromatics, comprising the steps of
a) acidifying the process wastewater by adding acid to a pH below 5, which forms an organic phase which separates out, an acidic aqueous phase and a gaseous NOₓ-containing phase, and
b) removing the gaseous NOₓ-containing phase.

2. The process according to claim 1, wherein acidification in step a) is accomplished using sulfuric acid from the workup of the aqueous sulfuric acid-containing phase obtained in the nitration.

3. The process according to claim 1 or 2, wherein acidification in step a) is accomplished using concentrated sulfuric acid from the workup of the aqueous sulfuric acid-containing phase obtained in the nitration.

4. The process according to any of claims 1 to 3, wherein the NOₓ formed in the course of acidification in step a) are stripped out of the organic and acidic aqueous phases by an inert gas and converted to the gaseous phase.

5. The process according to any of claims 1 to 4, wherein the NOₓ formed in the course of acidification in step a) are stripped out of the organic and acidic aqueous phases by nitrogen, mixtures of nitrogen and oxygen, air and/or carbon dioxide and converted to the gaseous phase.

6. The process according to any of claims 1 to 5, wherein the alkaline process wastewater originates from the scrubbing of the crude product from the nitration with aqueous alkali metal carbonate and/or alkali metal hydrogencarbonate solution, and the nitrogen oxides are stripped out of the organic and acidic aqueous phases by the carbon dioxide formed in the course of acidification of the alkaline process wastewater and converted to the gaseous phase.

7. The process according to any of claims 1 to 6, wherein the aromatic compounds to be nitrated is benzene, toluene, xylene, chlorobenzene and/or dichlorobenzene.

8. The process according to any of claims 1 to 7, wherein the alkaline process wastewater is acidified at temperatures of 20 to 90°C, preferably of 55 to 70°C.

9. The process according to any of claims 1 to 8, wherein step b) is performed before the further treatment of the organic and aqueous phases.

10. The process according to any of claims 1 to 9, wherein the acidic aqueous phase is extracted with a solvent after removal of the organic phase.

11. The process according to any of claims 1 to 10, wherein the acidic aqueous phase, after removal of the organic phase, optionally after extraction with a solvent, is sent to an ozonolysis, a thermolysis and/or a biological wastewater treatment.

12. The process according to any of claims 1 to 11, wherein step c) is performed after step b) and involves further processing of the NOₓ present in the gaseous phase removed to give nitric acid.

13. The process according to claim 12, wherein the NOₓ-containing phase removed is supplied in step c), without prior purification and removal of other gas constituents, directly to an absorption column for nitric acid production.

14. The process according to claim 12 or 13, wherein the nitric acid obtained in step c) is recycled into the nitration of the aromatic compounds.

15. The process according to any of claims 1 to 11, wherein the NOₓ-containing phase removed after step b) is sent to an incineration.

## Revendications

1. Procédé pour le traitement d'eaux usées alcalines de procédé, provenant de la nitration de composés aromatiques en mononitroaromatiques, dinitroaromatiques et trinitrioaromatiques, comprenant les étapes consistant à
a) acidifier l'eau usée de procédé par addition d'un acide à un pH inférieur à 5, avec formation d'une phase organique se séparant, d'une phase aqueuse acide et d'une phase gazeuse contenant NOₓ, et
b) évacuer la phase gazeuse contenant NOₓ.

2. Procédé selon la revendication 1, **caractérisé en ce que** pour l'acidification dans l'étape a), on utilise de l'acide sulfurique provenant du traitement de la phase aqueuse contenant de l'acide sulfurique produite lors de la nitration.

3. Procédé selon la revendication 1 ou 2, **caractérisé en ce que** pour l'acidification dans l'étape a), on utilise de l'acide sulfurique concentré provenant du traitement de la phase aqueuse contenant de l'acide sulfurique produite lors de la nitration.

4. Procédé selon l'une quelconque des revendications 1 à 3, **caractérisé en ce que** les NOₓ formés lors de l'acidification dans l'étape a) sont extraits par entraînement par un gaz inerte de la phase organique et de la phase aqueuse acide et transférés dans la phase gazeuse.

5. Procédé selon l'une quelconque des revendications 1 à 4, **caractérisé en ce que** les NOₓ formés lors de l'acidification dans l'étape a) sont extraits par entraînement par de l'azote, des mélanges d'azote et d'oxygène, de l'air et/ou du dioxyde de carbone de la phase organique et de la phase aqueuse acide et transférés dans la phase gazeuse.

6. Procédé selon l'une quelconque des revendications 1 à 5, **caractérisé en ce que** l'eau usée alcaline de procédé provient du lavage du produit brut de la nitration par une solution aqueuse de carbonate de métal alcalin et/ou d'hydrogénocarbonate de métal alcalin et les oxydes d'azote sont extraits par entraînement par le dioxyde de carbone formé lors de l'acidification de l'eau usée alcaline de la phase organique et de la phase aqueuse acide et sont transférés dans la phase gazeuse.

7. Procédé selon l'une quelconque des revendications 1 à 6, **caractérisé en ce qu'**il s'agit, pour le composé aromatique à nitrer, de benzène, de toluène, de xylène, de chlorobenzène et/ou de dichlorobenzène.

8. Procédé selon l'une quelconque des revendications 1 à 7, **caractérisé en ce que** l'eau usée alcaline de procédé est acidifiée à des températures de 20 à 90°C, de préférence de 55 à 70°C.

9. Procédé selon l'une quelconque des revendications 1 à 8, **caractérisé en ce que** l'étape b) est réalisée avant le traitement ultérieur de la phase organique et de la phase aqueuse.

10. Procédé selon l'une quelconque des revendications 1 à 9, **caractérisé en ce que** la phase aqueuse acide est extraite avec un solvant après la séparation de la phase organique.

11. Procédé selon l'une quelconque des revendications 1 à 10, **caractérisé en ce que** la phase aqueuse acide est introduite après la séparation de la phase organique, le cas échéant après extraction avec un solvant, dans une ozonolyse, une thermolyse et/ou une épuration biologique des eaux usées.

12. Procédé selon l'une quelconque des revendications 1 à 11, **caractérisé en ce que** l'étape c) est réalisée après l'étape b), les NOₓ contenus dans la phase gazeuse évacuée dans l'étape c) étant transformés en acide nitrique.

13. Procédé selon la revendication 12, **caractérisé en ce que** la phase contenant NOₓ évacuée dans l'étape c) est introduite sans purification ni séparation préalables d'autres constituants gazeux, directement dans une colonne d'absorption d'une préparation d'acide nitrique.

14. Procédé selon la revendication 12 ou 13, **caractérisé en ce que** l'acide nitrique contenu dans l'étape c) est recyclé dans la nitration des composés aromatiques.

15. Procédé selon l'une quelconque des revendications 1 à 11, **caractérisé en ce que** la phase contenant NOₓ évacuée après l'étape b) est introduite dans une combustion.
